# EUROPEAN PATENT APPLICATION

(11) **EP 4 840 939 A1**
(43) Date of publication of application: **18.11.2026**
(21) Application number: 25738778.7
(22) Date of filing: 08.01.2025
(51) Int. Cl.: C12N 5/0775, C12N 5/10

(54) **METHOD FOR ISOLATING SINGLE MESENCHYMAL STEM CELL**

(30) Priority: 12.01.2024 JP 2024003123
(71) Applicant: KANEKA CORPORATION, Osaka-shi, Osaka 530-8288 (JP)
(72) Inventor: HAYASHI, Masahiro, Kobe-shi, Hyogo 650-0047 (JP)
(74) Representative: Hoffmann Eitle
(86) International application number: PCT/JP2025/000355
(87) International publication number: WO 2025/150510

(57) **Abstract**

The present invention addresses the problem of providing a new method for efficiently isolating a mesenchymal stem cell as a single cell and efficiently proliferating the single cell. Provided is a method for isolating a single mesenchymal stem cell from a cell population comprising mesenchymal stem cells, comprising: a first culture step of culturing the cell population in a first culture medium comprising human platelet lysate, a collection step of collecting the cell population after the first culture step, and an isolation step of isolating a single mesenchymal stem cell from the cell population after the collection step.

## Description

### Technical Field

The present invention relates to a method for isolating a single mesenchymal stem cell, a method for producing a single mesenchymal stem cell, and the like.

### Background Art

A pluripotent stem cell is a cell having the ability to differentiate into various somatic cells. Examples of the pluripotent stem cell include an iPS cell obtained by introducing a reprogramming factor into a somatic cell, an ES cell derived from an inner cell mass, and a mesenchymal stem cell that is a somatic stem cell.

Among these, the mesenchymal stem cell is known to have the ability to differentiate into an osteoblast, a chondrocyte, an adipocyte, and the like, and has been revealed to exist in various tissues such as bone marrow, fat, dental pulp, and a fetal appendage (a placenta, an umbilical cord, a fetal membrane, and the like) (Patent Literature 1). The mesenchymal stem cell is being put into practical use as a regenerative medicine material based on the pluripotency thereof. **In** practice, clinical applications to a wide range of diseases such as acute graft-versus-host disease (GVHD), Crohn's disease, spinal cord injury, and knee osteoarthritis are being advanced based on the fact that a mesenchymal stem cell has immunosuppressive ability. Furthermore, since the mesenchymal stem cell has low immunogenicity and can be allografted, the mesenchymal stem cell is also useful as a regenerative medicine material for ex vivo gene therapy.

**In** order to isolate a mesenchymal stem cell from a mesenchymal tissue or a fetal appendage and use the cell, it is necessary to remove cells other than the mesenchymal stem cell that are comprised by the same tissue. For example, bone marrow fluid comprises many hematopoietic stem cells in addition to mesenchymal stem cells, and fat comprises many adipocytes in addition to mesenchymal stem cells. Accordingly, it is desirable to remove these hematopoietic stem cells and adipocytes. Additionally, an amniotic membrane, which is a kind of fetal appendage, is attracting attention as a promising biological tissue comprising many mesenchymal stem cells. However, since the amniotic membrane comprises many epithelial cells in addition to mesenchymal stem cells, it is desirable to remove the epithelial cells.

Additionally, from the viewpoint of cell quality control, it is further desirable to isolate a mesenchymal stem cell as a single cell, expand and culture the single cell, and produce a highly homogeneous cell population. For example, in a case where a mesenchymal stem cell transformed with a plasmid vector or a viral vector is used for treatment, a difference in quality can occur between cells based on a difference in the number of introduced copies of the vector, a genome insertion position, or the like. **In** order to obtain a highly homogeneous cell population, it is necessary to isolate a single cell from mesenchymal stem cells having a gene introduced thereinto, and expand and culture the single cell.

Accordingly, there is a demand for a new method for efficiently isolating a mesenchymal stem cell as a single cell and efficiently proliferating the single cell.

### Citation List

### Patent Literature

Patent Literature 1: WO2015/025810

### Summary of Invention

### Technical Problem

An object of the present invention is to provide a new method for efficiently isolating a mesenchymal stem cell as a single cell and efficiently proliferating the single cell.

### Solution to Problem

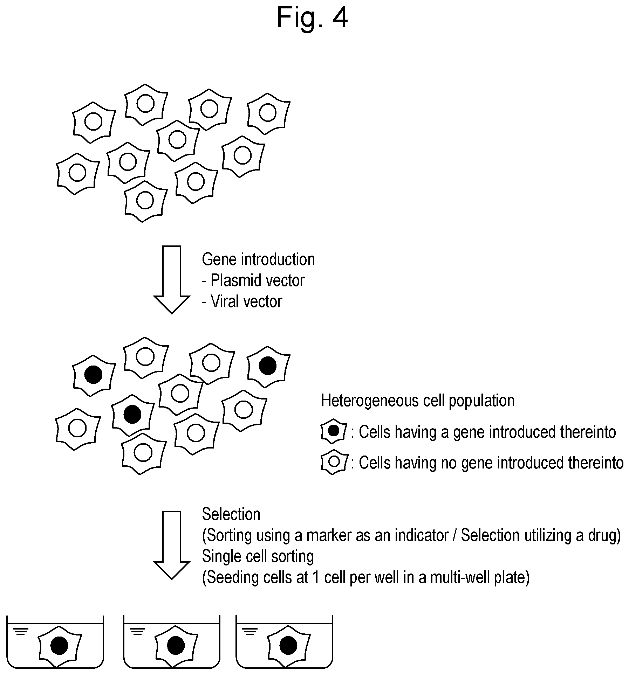

**In** single cell cloning in which a single cell is isolated from a cell population comprising a plurality of cells and is proliferated, a gene is introduced if necessary, and then, the cells are isolated one by one using a cell sorter or the like, and one cell is seeded and cultured in each well in a multi-well plate, whereby a cell population derived from the single cell is obtained (Figure 4).

**In** order to isolate a mesenchymal stem cell as a single cell, the present inventors cultured a mesenchymal stem cell collected from an amniotic membrane, in a culture medium comprising a commonly used fetal bovine serum (FBS). After isolating and seeding one cultured amniotic membrane mesenchymal stem cell into each well in a 96-well plate using a cell sorter, the present inventors attempted to proliferate the cells by further culturing. However, after the amniotic membrane mesenchymal stem cell cultured in the culture medium comprising FBS was isolated as a single cell, cell proliferation was not observed in any of the 96 wells.

Next, the present inventors cultured an amniotic membrane mesenchymal stem cell in a culture medium comprising human platelet lysate (hPL) instead of a fetal bovine serum (FBS), and isolated and cultured one cell in each well in a 96-well plate using a cell sorter. As a result, cell proliferation of the amniotic membrane mesenchymal stem cell was observed in nearly half of the wells.

Furthermore, the present inventors performed a similar experiment using a bone marrow mesenchymal stem cell as a cell of interest. As a result of culturing a bone marrow mesenchymal stem cell in a culture medium comprising FBS, then using a cell sorter to isolate one cell in each well in a 96-well plate, and culturing the cells, cell proliferation was observed only in 20 out of 96 wells. On the other hand, in a case where a bone marrow mesenchymal stem cell was cultured in a culture medium comprising hPL and then isolated and cultured one cell at a time, cell proliferation was observed in 45 out of 96 wells.

The above results have revealed that mesenchymal stem cells having proliferative ability can be efficiently obtained as single cells by culturing the cells before and/or after cell sorting, in a culture medium comprising an hPL. The present invention is based on the above findings and provides the following.
(1) A method for isolating a single mesenchymal stem cell from a cell population comprising mesenchymal stem cells, comprising:
   a first culture step of culturing the cell population in a first culture medium comprising human platelet lysate;
   a collection step of collecting the cell population after the first culture step; and
   an isolation step of isolating a single mesenchymal stem cell from the cell population after the collection step.
(2) The method of (1), wherein the cell population is derived from amniotic membrane or bone marrow.
(3) The method of (1) or (2), wherein the first culture medium comprises 3 v/v% or more of the human platelet lysate.
(4) The method of (3), wherein the first culture medium comprises 3 to 20 v/v% of the human platelet lysate.
(5) The method of any one of (1) to (4), wherein the first culture medium further comprises heparin or a heparin substitute.
(6) The method of any one of (1) to (5), wherein the first culture medium is a basic culture medium or a serum-free culture medium.
(7) The method of any one of (1) to (6), wherein the collection step comprises collecting the cell population from the first culture medium.
(8) The method of any one of (1) to (7), wherein dead cells or living cells are labeled after the collection step, and only the living cells are isolated in the isolation step.
(9) The method of any one of (1) to (8), further comprising an introduction step of introducing a gene expression vector into the cell population before the isolation step.
(10) The method of (9), wherein the gene expression vector comprises a marker gene.
(11) The method of (9) or (10), wherein the introduction step is performed before the first culture step.
(12) The method of (10) or (11), wherein the isolation step comprises isolating the single mesenchymal stem cell expressing a marker protein encoded by the marker gene, using the expression of the marker protein as a marker.
(13) The method of any one of (10) to (12), wherein the marker gene is an antibiotic resistance gene that confers resistance to an antibiotic.
(14) The method of any one of (1) to (13), further comprising a second culture step of culturing the single mesenchymal stem cell after the isolation step in a second culture medium that does not comprise other mesenchymal stem cells.
(15) The method of (14), wherein the second culture medium comprises human platelet lysate.
(16) The method of (14) referring to (13) or of (15) referring to (13) and (14), wherein the second culture medium comprises the antibiotic.
(17) A method for producing a single mesenchymal stem cell, comprising producing the single mesenchymal stem cell using the method of (1) to (16).
(18) A method for producing a population of mesenchymal stem cells derived from a single mesenchymal stem cell, comprising producing the population of mesenchymal stem cells by proliferating the single mesenchymal stem cell in the second culture step using the method of any one of (14) to (16).
(19) A method for culturing a single mesenchymal stem cell, comprising:
   a first culture step of culturing a cell population comprising mesenchymal stem cells in a first culture medium;
   a collection step of collecting the cell population after the first culture step;
   an isolation step of isolating a single mesenchymal stem cell from the cell population after the collection step; and
   a second culture step of culturing the single mesenchymal stem cell after the isolation step in a second culture medium that does not comprise other mesenchymal stem cells,
   wherein the second culture medium comprises human platelet lysate.

The present specification encompasses the disclosure of Japanese Patent Application No. 2024-003123 that serves as the basis of the priority of the present application.

### Advantageous Effects of Invention

The present invention provides a new method for efficiently isolating a mesenchymal stem cell as a single cell, and efficiently proliferating the single cell.

### Brief Description of Drawing

[Figure 1] Figure 1 schematically shows an outline of an experiment for identifying wells comprising proliferating cells after isolating mesenchymal stem cells as single cells, following FBS culture or hPL culture.
[Figure 2] Figure 2 shows the result of counting the number of wells comprising proliferating cells in 96 wells after isolating bone marrow mesenchymal stem cells (Bone marrow MSCs in the figure) or amniotic membrane mesenchymal stem cells (Amniotic membrane MSCs in the figure) as single cells, following FBS culture or hPL culture.
[Figure 3] Figure 3 shows the number of doublings of bone marrow mesenchymal stem cells and amniotic membrane mesenchymal stem cells during FBS culture over a long period of time.
[Figure 4] Figure 4 schematically shows a method for isolating single cells into which a marker gene is introduced in an embodiment of the present invention.

### Description of Embodiments

### 1. Method for isolating single mesenchymal stem cell

### 1-1. Overview

A first aspect of the present invention is a method for isolating a single mesenchymal stem cell from a cell population comprising mesenchymal stem cells (hereinafter abbreviated as "isolation method" in some cases). The isolation method of the present aspect comprises, as essential steps, a first culture step of culturing a cell population comprising mesenchymal stem cells in a first culture medium comprising human platelet lysate, a collection step of collecting the cell population, and an isolation step of isolating a single mesenchymal stem cell. The mesenchymal stem cell isolated by the isolation method of the present aspect efficiently proliferates under a culture condition of a single cell. Accordingly, a highly homogeneous population of mesenchymal stem cells can be efficiently produced.

### 1-2. Definitions of terms

The following terms frequently used in the present specification are defined.

As used herein, "fetal appendage" refers to a tissue or an organ that is other than a fetal body in a uterus, and supports fetal development, and specifically refers to a fetal membrane, a placenta, an umbilical cord, and amniotic fluid. "Fetal membrane" is a gestational sac comprising fetal amniotic fluid, consisting of an amniotic membrane, a chorion, and a decidua from the inside. "Amniotic membrane" refers to a transparent thin membrane poor in blood vessels in the innermost layer of a fetal membrane. An inner layer of an amniotic membrane (also called an epithelial cell layer) is covered with a single layer of epithelial cells having a secretory function, and secretes amniotic fluid. An outer layer of an amniotic membrane (also called an extracellular matrix layer, corresponding to a stroma) comprises a mesenchymal stem cell.

As used herein, "mesenchymal stem cell" refers to a stem cell that satisfies (i) exhibiting adherence to the surface of a plastic culture vessel under culture conditions in a standard culture medium (a culture medium obtained by adding serum, a serum replacement reagent, or a growth factor to a basic culture medium), and (ii) being positive for surface antigens CD105, CD73, and CD90, and being negative for CD45. As used herein, "mesenchymal stem cell" is also referred to as "MSC", and is synonymous with "mesenchymal stromal cell". A biological species from which a mesenchymal stem cell derives is not particularly limited as long as it is a biological species having the mesenchymal stem cell, and may be, for example, a fish, a bird, or a mammal. The mesenchymal stem cell may be, for example, a mouse cell, a chimpanzee cell, or a human cell. Additionally, examples of the origin of the mesenchymal stem cell include a bone marrow, a hematopoietic stem cell, an umbilical cord blood, an umbilical cord, an amniotic membrane, an amniotic fluid, a placental villus, a nerve, an adipose tissue, a pancreas, a synovium, a dental pulp, a deciduous tooth, a sperm, a testis, a cornea, and the like, but are not limited thereto. Additionally, the mesenchymal stem cell may be an immortalized mesenchymal stem cell line.

As used herein, "cell population comprising mesenchymal stem cells" is a cell population comprising mesenchymal stem cells derived from, for example, any of the above-described tissues (for example, an amniotic membrane or a bone marrow). The form of the cell population comprising mesenchymal stem cells is not particularly limited, and may be, for example, a cell pellet, a cell aggregate, a cell floating liquid, a cell suspension, or the like.

As used herein, "amniotic membrane mesenchymal stem cell" refers to a mesenchymal stem cell derived from an amniotic membrane. **In** the present specification, the amniotic membrane mesenchymal stem cell is also referred to as "amniotic membrane MSC".

Additionally, in the present specification, "bone marrow mesenchymal stem cell" refers to a mesenchymal stem cell derived from a bone marrow. **In** the present specification, the bone marrow mesenchymal stem cell is also referred to as "bone marrow MSC".

As used herein, "culture medium" is not limited as long as it is a culture medium capable of maintaining a cell such as a mesenchymal stem cell as a living cell. For example, the culture medium may be a culture medium known in the art and commonly used in cell culture. The culture medium may be any of a basic culture medium, a serum-free culture medium, a low-serum culture medium, and a serum-supplemented culture medium, but is usually a basic culture medium (for example, a standard cell culture medium) or a serum-free culture medium, and can be prepared by adding another component if necessary. The "standard cell culture medium" as used herein refers to a highly versatile basic culture medium mainly used for culturing various types of cells derived from mammals. Specific examples of a culture medium that may be used include, but are not particularly limited to, a BME medium, a BGJb medium, a CMRL1066 medium, a Glasgow MEM medium, an Improved MEM Zinc Option medium, an IMDM medium (Iscove's Modified Dulbecco's Medium), a Medium 199 medium, an Eagle MEM medium, an αMEM (Alpha Modification of Minimum Essential Medium Eagle) medium, an MEM-α (Minimum Essential Medium α) medium, a DMEM medium (Dulbecco's Modified Eagle's Medium), a Ham's F10 medium, a Ham's F12 medium, an RPMI 1640 medium, a Fischer's medium, and a mixed culture medium thereof (for example, a DMEM/F12 medium (Dulbecco's Modified Eagle's Medium/Nutrient Mixture F-12 Ham)). Additionally, various commercially available serum-free culture mediums may be used. Examples of the another component added to a basic culture medium include albumin, a blood-derived component, and a growth factor.

As used herein, "single cell" means one cell comprising no other cell at all. In the present specification, a single cell is also referred to as "single cell".

As used herein, "single cell cloning" means isolating a single cell (that is, a single cell) having proliferative ability from a cell population comprising two or more cells, and the isolated single cell is cultured thereafter. For example, in a case where single cell cloning is performed on a mesenchymal stem cell, one isolated mesenchymal stem cell is cultured in a culture medium not comprising any other cell such as another mesenchymal stem cell.

As used herein, "proliferative ability" refers to the ability of a cell to increase the number of cells by cell division. For example, in a case where a mesenchymal stem cell is cultured as a single cell, a case exhibiting proliferative ability and a case exhibiting no proliferative ability are observed. **In** a case where a single cell of a mesenchymal stem cell exhibits proliferative ability, the mesenchymal stem cell has the ability to produce two or more cells by cell division and to form two or more cells derived from the cell.

### 1-3. Method

The isolation method of the present aspect comprises a first culture step, a collection step, and an isolation step as essential steps, and may comprise a repeating step, an introduction step, and/or a second culture step as optional steps. Hereinafter, each step in the isolation method of the present aspect will be specifically described.

### (First culture step)

**In** the method of the present invention, "first culture step" is a step of culturing a cell population comprising mesenchymal stem cells in a first culture medium comprising human platelet lysate. A culture method in this step is not limited, and may be, for example, adherent culture, suspension culture, or spheroid culture. Since a mesenchymal stem cell is an adherent cell, the culture method in this step is preferably adherent culture. However, suspension culture in which a microcarrier having a mesenchymal stem cell attached thereto is suspended and cultured, or spheroid culture in which a mesenchymal stem cell is spheroidized and cultured may be used.

The "first culture medium" used in this step is a culture medium in which human platelet lysate is added at an arbitrary concentration as an essential component to the above-described "culture medium", and may comprise another component if necessary. **In** this first culture medium, a single cell immediately before the below-described isolation step can be cultured.

As used herein, "platelet lysate" refers to a lysate of blood-derived platelets that serves as an additive for cell culture, comprising large amounts of a plurality of growth factors and chemokines as components thereof. An animal species from which a platelet lysate derives is not particularly limited in the present specification, but is preferably a human. In the present specification, a human-derived platelet lysate (human platelet lysate) is often abbreviated as "hPL". Examples of a growth factor that may be comprised in the human platelet lysate include platelet derived growth factor isoforms (PDGF-AA, -AB, and -BB), transforming growth factor-β (TGF-β), insulin-like growth factor-1 (IGF-1), brain derived neurotrophic factor (BDNF), vascular endothelial growth factor (VEGF), epidermal growth factor (EGF), basic fibroblast growth factor (bFGF or FGF-2), hepatocyte growth factor (HGF), connective tissue growth factor (CTGF), and morphogenetic proteins-2, -4, and -6 (BMP-2, -4, and -6). Additionally, examples of a chemokine that may be comprised in the above human platelet lysate include interleukin-8 (IL-8), neutrophil-activating peptide-2 (NAP-2), regulated on activation, normal T cell expressed and secreted (TANTES), monocyte chemotactic proteins-1 and -3 (MCP-1 and -3), macrophage inflammatory proteins-1 alpha (MIP-1α), and beta-thromboglobulin.

A method for producing human platelet lysate used in the method of the present invention is not particularly limited. The platelet lysate is typically produced by repeatedly freezing and thawing platelets extracted from whole blood, followed by purification to remove cell fragments and the like, but the production method is not particularly limited, and may be any method. For a specific preparation method, reference may be made to literature such as Schallmoser k et al., Methods Mol Biol. 2013;946:349-62, and the like.

The human platelet lysate used in the present invention is preferably subjected to an inactivation and/or sterilization treatment of bacteria and viruses.

In the method of the present invention, a commercially available product of human platelet lysate may be used. Examples of a commercially available product of human platelet lysate include Stemulate (manufactured by Cook Regentec LLC), PLTMax (manufactured by Mill Creek Life Science, LLC), UltraGRO (manufactured by AventaCell BioMedical Corp.), and PLUS (manufactured by Compass Biomedical, Inc.).

**In** the method of the present invention, a clarified human platelet lysate may be used. Clarification as mentioned herein refers to a treatment of removing an insoluble fraction generated in long-term storage or freezing and thawing from human platelet lysate. Examples of a clarification method include centrifugation, filtration, precipitation, and sedimentation (for example, natural sedimentation), but are not limited thereto as long as the method enables an insoluble fraction to be removed.

The concentration of the human platelet lysate in the first culture medium is not particularly limited. The first culture medium comprises human platelet lysate of, for example, 0.1 v/v% or more, 0.2 v/v% or more, 0.3 v/v% or more, 0.4 v/v% or more, 0.5 v/v% or more, 0.6 v/v% or more, 0.7 v/v% or more, 0.8 v/v% or more, 0.9 v/v% or more, 1 v/v% or more, 1.2 v/v% or more, 1.5 v/v% or more, 2 v/v% or more, 2.5 v/v% or more, 3 v/v% or more, 3.5 v/v% or more, 4 v/v% or more, 4.5 v/v% or more, or 5 v/v% or more, and/or 40 v/v% or less, 35 v/v% or less, 30 v/v% or less, 25 v/v% or less, 20 v/v% or less, 15 v/v% or less, or 10 v/v% or less. **In** the present specification, in a case where the concentration of human platelet lysate is expressed in units of "v/v%", the concentration indicates the dilution ratio by volume of the human platelet lysate (amount of hPL liquid before dilution/amount of liquid after dilution), and more specifically, indicates a volume ratio at which a crude platelet lysate or a commercially available platelet lysate is diluted with a culture medium such as a first culture medium. Note that, as a commercially available platelet lysate, for example, human platelet lysate having a total protein concentration of 40 to 80 mg/mL may be diluted and used.

The concentration of the human platelet lysate in the first culture medium may be expressed in terms of the dry weight of human platelet lysate. The concentration of the human platelet lysate in the first culture medium may be, for example, 0.06 mg/mL or more, 0.3 mg/mL or more, 0.6 mg/mL or more, 1.2 mg/mL or more, 1.8 mg/mL or more, or 3 mg/mL or more, and/or 24 mg/mL or less, 21 mg/mL or less, 18 mg/mL or less, 15 mg/mL or less, 12 mg/mL or less, 9 mg/mL or less, or 6 mg/mL or less in terms of dry weight.

Additionally, the concentration of the human platelet lysate in the first culture medium can also be specified by the human platelet lysate protein concentration per 1 mL of the first culture medium. For example, the human platelet lysate protein concentration per 1 mL of the first culture medium may be 0.02 mg/mL to 32 mg/mL, 0.04 mg/mL to 28 mg/mL, 0.2 mg/mL to 24 mg/mL, 0.4 mg/mL to 20 mg/mL, 0.8 mg/mL to 16 mg/mL, 1.2 mg/mL to 12 mg/mL, 1.6 mg/mL to 8 mg/mL, or 2 mg/mL to 4 mg/mL, and may be, for example, 3 mg/mL.

The first culture medium may comprise another component in addition to human platelet lysate that is an essential component. For example, the first culture medium may comprise an albumin, a blood-derived component, a growth factor, and/or the like in addition to human platelet lysate. **In** a case where the first culture medium comprises an albumin in addition to human platelet lysate, the concentration of the albumin may be, for example, 0.05% by mass to 5% by mass. Additionally, examples of a blood-derived component include various serums (animal-derived serums such as a fetal bovine serum (FBS and FCS) and a human serum) and/or plasma. **In** a case where the first culture medium comprises a blood-derived component in addition to human platelet lysate, the concentration of the blood-derived component may be, for example, 2% by volume to 40% by volume, 3% by volume to 30% by volume, or 5% by volume to 20% by volume, and may be, for example, 10% by volume. **In a** case where the first culture medium comprises a growth factor in addition to human platelet lysate, a reagent (an anticoagulant such as heparin, a gel, a polysaccharide, or the like) for stabilizing the growth factor in a culture medium may be further added in addition to the growth factor. Examples of a growth factor that may be used include, but are not particularly limited to, a fibroblast growth factor (FGF), an epidermal growth factor (EGF), a transforming growth factor (TGF), a vascular endothelial growth factor (VEGF), a platelet-derived growth factor (PDGF), and factors belonging to families thereof.

**In** one embodiment, the first culture medium further comprises heparin or a heparin substitute. For example, the human platelet lysate comprised in the first culture medium may comprise heparin or a heparin substitute. Heparin has an anticoagulant activity and can stabilize human platelet lysate in a culture medium. As used herein, "heparin substitute" is any substance having an anticoagulant activity similar to heparin. Specific types of a heparin substitute are known in the art, and specific examples thereof include fucoidan, sulfated fucan, and D-phenylalanyl-L-prolyl-L-arginine chloromethyl ketone (PPACK). The concentration of heparin or a heparin substitute in the first culture medium is, for example, 0.1 U/mL or more, 1 U/mL or more, or 2 U/mL or more, and is, for example, 2 U/mL.

A culture temperature in this step is not limited as long as it is a temperature at which a mesenchymal stem cell can grow. For example, a culture in this step may be performed at 30 to 42°C, 32 to 40°C, 35 to 39°C, or 36 to 38°C, for example, 37°C.

A culture time in this step is not particularly limited, and may be, for example, 1 hour or more, 2 hours or more, 4 hours or more, 12 hours or more, 24 hours or more, 2 days or more, or 3 days or more, and/or 2 weeks or less, 1 week or less, 6 days or less, 5 days or less, or 4 days or less. For example, a culture may be performed until mesenchymal stem cells become subconfluent.

### (Collection step)

In the method of the present invention, "collection step" is a step of collecting a cell population. A collection method in this step can be appropriately selected according to the kind of a culture method in the first culture step. For example, in a case where the culture method in the first culture step is adherent culture, a mesenchymal stem cell can be collected by detaching cells after adherent culture from a culture vessel. Additionally, in a case where the culture method in a first culture step uses a microcarrier, a cell may be detached from a microcarrier, and in a case of spheroid culture, a mesenchymal stem cell may be collected by dispersing a spheroid.

As used herein, "detaching" refers to a cell physically separating from a surface of a culture substrate such as a culture vessel and a microcarrier. A cell may be detached in units of cell masses or single cells. Examples of the detachment include physical detachment (for example: mechanical detachment performed by rubbing a vessel surface using a cell scraper or the like; splashing; a method using rocking and vibration, and ultrasonic treatment); enzymatic detachment using an enzyme such as a protease; chemical detachment using a compound such as a chelating agent; and any combination thereof.

A method for collecting a mesenchymal stem cell after detachment from a culture substrate such as a culture vessel and a microcarrier is not limited, and for example, a cell suspension comprising a detached mesenchymal stem cell may be collected by suction or decantation.

The number of mesenchymal stem cells collected in this step is not limited, but is, for example, 1 × 10¹ or more, 1 × 10² or more, 1 × 10³ or more, 1 × 10⁴ or more, 1 × 10⁵ or more, or 1 × 10⁶ or more. Additionally, the viability of cells collected in this step is not limited, but is, for example, 50% or more, 70% or more, 80% or more, 90% or more, or 95% or more.

**In** one embodiment, this step collects a cell population from the first culture medium used in the above-described first culture step. According to this embodiment, a mesenchymal stem cell immediately after culturing in the first culture medium is isolated in the below-described isolation step.

### (Repeating step)

**In** the method of the present aspect, "repeating step" is an optional step of repeating the steps from the above-described first culture step to the collection step. The number of repetitions of this step is not limited. The method of the present aspect comprises, for example, one, two, three, or four or more repeating steps.

### (Introduction step)

In the method of the present invention, "introduction step" is an optional step of introducing a gene expression vector into a cell population before the below-described isolation step.

As used herein, "gene expression vector" refers to a vector comprising a gene or gene fragment of interest in an expressible state, and encompassing an expression unit capable of controlling expression of the gene or the like. The gene expression vector may be a plasmid vector or a viral vector. The gene expression vector may comprise a labeling gene (a selection marker gene) if necessary.

A gene or gene fragment of interest comprised in the gene expression vector is not particularly limited, and may be a gene sequence encoding a protein of interest or a fragment thereof or an RNA molecule of interest. A protein of interest or a fragment thereof is not particularly limited, and may be appropriately selected according to the purpose. Specific examples include gene sequences encoding IL-10 (Interleukin-10), HGF (Hepatocyte Growth Factor), VEGF (Vascular Endothelial Growth Factor), BDNF (Brain Derived Neurotrophic Factor), and miRNA (microRNA) for the purpose of functional enhancement.

As used herein, "expressible state" means that a gene to be expressed or the like is arranged in a downstream region of a promoter under control of the promoter.

The plasmid vector may be, for example, a commercially available expression vector for a mammalian cell, such as a pCI vector and a pSI vector manufactured by Promega Corporation, or a shuttle vector replicable in both mammalian cells and bacteria such as *Escherichia coli.*

Examples of a viral vector that may be used include viral vectors such as a retroviral vector (including an oncoretroviral vector, a lentiviral vector, and a pseudotyped vector), an adenoviral vector, an adeno-associated viral (AAV) vector, a simian viral vector, a vaccinia viral vector, a Sendai viral vector, an Epstein-Barr viral (EBV) vector, and an HSV vector. A viral vector lacking replication ability so as not to self-replicate in an infected cell may be used.

A method for introducing a gene expression vector into a cell population before the isolation step in this step is not particularly limited. For example, a gene introducing method (transformation method) known in the art described in Green & Sambrook, 2012, Molecular Cloning: A Laboratory Manual Fourth Ed., Cold Spring Harbor Laboratory Press, Cold Spring Harbor, New York, and the like may be used. Specific examples include a heat shock method, a lipofection method, an electroporation method, a microinjection method, a calcium phosphate method, a DEAE dextran method, introduction using a cationic lipid, introduction using a cationic polymer (for example, polyethyleneimine (PEI)), introduction using a nanoparticle, and introduction using a virus, particle bombardment.

As used herein, "labeling gene" or "marker gene" refers to a gene encoding a labeling protein (marker protein), which is also referred to as a selection marker or a reporter protein. "Labeling protein" or "marker protein" is not limited as long as it can label and/or select a cell having a gene expression vector introduced thereinto, based on the activity thereof. Examples include a drug (for example, antibiotic) resistance protein, a chromoprotein, a fluorescent protein, and a luminescent protein.

This step may be performed before the first culture step or after the first culture step as long as it is before the isolation step. For example, in a case where this step is performed before the first culture step, a mesenchymal stem cell comprising a gene expression vector can be selected in a culture medium (for example, the first culture medium used for a culture before the below-described isolation step, or the second culture medium used for a culture after the isolation step) comprising a drug (for example, antibiotic) corresponding to a gene encoding a drug resistance protein in the gene expression vector. Additionally, in a case where this step is performed after the first culture step, a mesenchymal stem cell comprising a gene expression vector may be selected by fluorescence detection using a cell sorter or the like in the below-described isolation step, based on a gene encoding a fluorescent protein in the gene expression vector.

### (Isolation step)

In the method of the present invention, "isolation step" is a step of isolating a single mesenchymal stem cell from the cell population after the collection step.

As used herein, "isolating a single mesenchymal stem cell" means it is sufficient that a single mesenchymal stem cell is separated from other mesenchymal stem cells, and it may or may not be separated from cell types other than mesenchymal stem cells; however, it is more preferable that the single mesenchymal stem cell is separated from living cells (for example, cells having proliferative ability) other than mesenchymal stem cells.

An isolation method used in this step is not limited as long as it is a method capable of separating mesenchymal stem cells after the above-described collection step into single cells. For example, by diluting a suspension comprising mesenchymal stem cells and dispensing the suspension into a multi-well plate such as a 96-well plate so as to achieve 1 cell/well, each cell can be sorted into each well. In a case where mesenchymal stem cells are labeled with a fluorescent substance or a dye, the cells can be sorted individually using a cell sorter or the like by flow cytometry with fluorescence or a dye used as an indicator. For labeling a mesenchymal stem cell, for example, a labeling gene (marker gene) may be introduced into a mesenchymal stem cell using a gene expression vector, or an antibody labeled with a fluorescent substance may be bound to a mesenchymal stem cell. Additionally, cells may be sorted individually using a micromanipulator, a micromesh filter, or the like.

In one embodiment, this step can isolate only a living cell by labeling a dead cell or a living cell after the above-described collection step. Examples of a labeling method include staining dead cells with a 7-AAD (7-Amino-Actinomycin D) staining solution or PI (Propidium Iodide).

**In** a case where a plurality of single mesenchymal stem cells are isolated by this step, the plurality of single mesenchymal stem cells can be sorted into a plurality of culture vessels or a plurality of wells of a multi-well plate. The shape of a culture vessel or a multi-well plate is not limited, and for example, by sorting a single cell into each well in a multi-well plate such as a 6-well plate, a 12-well plate, or a 96-well plate, a plurality of cells may be respectively isolated as single cells.

### (Second culture step)

**In** the method of the present invention, "second culture step" is an optional step of culturing a single cell of a mesenchymal stem cell obtained after the isolation step in the second culture medium comprising no other mesenchymal stem cell.

"Second culture medium" used in this step refers to a culture medium for culturing a single mesenchymal stem cell as a single cell. **In** this second culture medium, a single cell immediately after the above-described isolation step can be cultured.

As used herein, "culturing as a single cell" refers to culturing under conditions where only one cell is comprised in the same culture solution. The second culture medium used in the second culture step comprises no cell other than one mesenchymal stem cell obtained after the isolation step.

The specific constitution of the second culture medium conforms to the above-described "culture medium". For example, the second culture medium may be any of a basic culture medium, a serum-free culture medium, a low-serum culture medium, and a serum-supplemented culture medium. It is usually a basic culture medium (for example, a standard cell culture medium) or a serum-free culture medium, and may be a culture medium obtained by adding another component such as an albumin, a blood-derived component, or a growth factor to a basic culture medium.

In one embodiment, the second culture medium comprises human platelet lysate. The concentration of the human platelet lysate in the second culture medium is not particularly limited, and may be 0.1 v/v% or more, 0.2 v/v% or more, 0.3 v/v% or more, 0.4 v/v% or more, 0.5 v/v% or more, 0.6 v/v% or more, 0.7 v/v% or more, 0.8 v/v% or more, 0.9 v/v% or more, 1 v/v% or more, 1.2 v/v% or more, 1.5 v/v% or more, 2 v/v% or more, 2.5 v/v% or more, 3 v/v% or more, 3.5 v/v% or more, 4 v/v% or more, 4.5 v/v% or more, or 5 v/v% or more, and/or 40 v/v% or less, 35 v/v% or less, 30 v/v% or less, 25 v/v% or less, 20 v/v% or less, 15 v/v% or less, or 10 v/v% or less, and may be any of the above concentrations expressed in terms of dry weight or the above-described human platelet lysate protein concentration.

In another embodiment, the second culture medium comprises no human platelet lysate. For example, the second culture medium may be a basic culture medium or a serum-free culture medium optionally supplemented with a blood-derived component such as a serum (an animal-derived serum such as a fetal bovine serum (FBS and FCS), or a human serum). Thus, even in a case where the second culture medium comprises no human platelet lysate, as long as the first culture medium comprises human platelet lysate, a single cell of a mesenchymal stem cell having proliferative ability can be obtained with high efficiency.

In one embodiment, the second culture medium comprises an antibiotic. By performing a culture in this step in the presence of this antibiotic, a mesenchymal stem cell comprising a gene encoding an antibiotic resistance protein introduced in the above-described introduction step can be selected.

A time from a culture in the above-described first culture step to the start of a culture in this step is not particularly limited, but a culture in this step is performed, for example, within 24 hours, within 18 hours, within 12 hours, within 6 hours, within 4 hours, within 3 hours, within 2 hours, or within 1 hour from the culture in the first culture step.

### 1-4. Effect

The isolation method of the present invention makes it possible that a single mesenchymal stem cell having proliferative ability can be efficiently obtained by culturing the mesenchymal stem cell in a culture medium comprising hPL in the first culture step before the isolation step.

### 2. Method for producing mesenchymal stem cell population derived from single mesenchymal stem cell

### 2-1. Overview

A second aspect of the present invention is a method for producing a mesenchymal stem cell population derived from a single mesenchymal stem cell (hereinafter abbreviated as "production method" in some cases). The production method of the present aspect comprises producing a single cell based on isolating a single mesenchymal stem cell using the isolation method described in the above-described first aspect, and can efficiently produce a single mesenchymal stem cell or a cell population consisting of a plurality of mesenchymal stem cells derived from a single mesenchymal stem cell.

### 2-2. Method

The production method of the present aspect comprises producing a single mesenchymal stem cell using the method described in the above-described first aspect. Accordingly, the production method of the present aspect also comprises the above-described first culture step, collection step, and isolation step as essential steps, and may comprise the above-described repeating step, introduction step, and/or second culture step as optional steps. Since the constitution of each step conforms to the first aspect, a detailed description thereof is omitted here.

In one embodiment, the production method of the present aspect can produce a cell population consisting of a plurality of mesenchymal stem cells by proliferating a single mesenchymal stem cell in the second culture step using the method described in the first aspect comprising the above-described second culture step.

The present invention also provides: a single mesenchymal stem cell produced by the production method of the present aspect, a culture solution comprising the single mesenchymal stem cell, and a culture supernatant of the single mesenchymal stem cell; and a cell population consisting of mesenchymal stem cells derived from the single mesenchymal stem cell produced by the production method of the present aspect, a culture solution comprising the cell population, and a culture supernatant of the cell population.

Additionally, there is also provided a method for evaluating a mesenchymal stem cell into which an arbitrary foreign gene has been introduced, wherein the subject to be evaluated is a single mesenchymal stem cell produced by the production method of the present aspect, or a cell population consisting of mesenchymal stem cells derived from a single mesenchymal stem cell produced by the production method of the present aspect.

### 3. Method for culturing single mesenchymal stem cell

### 3-1. Overview

A third aspect of the present invention is a method for culturing a single mesenchymal stem cell (hereinafter abbreviated as "culture method" in some cases). The culture method of the present aspect is characterized by comprising: a first culture step of culturing a cell population comprising mesenchymal stem cells in a first culture medium, a collection step of collecting the cell population, an isolation step of isolating a single mesenchymal stem cell, and a second culture step of culturing the single mesenchymal stem cell after the isolation step in a second culture medium that does not comprise other mesenchymal stem cells, wherein the second culture medium comprises human platelet lysate. In the culture method of the present aspect, a single mesenchymal stem cell can be efficiently proliferated, and a highly homogeneous population of a mesenchymal stem cell can be efficiently produced.

### 3-2. Method

The culture method of the present aspect comprises a first culture step, a collection step, an isolation step, and a second culture step as essential steps, and may comprise a repeating step and/or an introduction step as optional steps.

The constitution of each step in the present aspect conforms to the first aspect. However, in the culture method of the present aspect, the first culture medium used in the first culture step may or may not comprise human platelet lysate, and the constitution excluding human platelet lysate conforms to the constitution of the first culture medium of the first aspect. For example, the first culture medium used in the first culture step in the present aspect may be constituted according to the second culture medium in the first aspect.

Additionally, in the culture method of the present aspect, the second culture medium used in the second culture step comprises human platelet lysate as an essential component, and is a culture medium supplemented with the human platelet lysate at an arbitrary concentration. The constitution other than the human platelet lysate conforms to the constitution of the second culture medium of the first aspect. For example, the second culture medium used in the second culture step in the culture method of the present aspect may be constituted according to the first culture medium in the first aspect.

**In** a further aspect of the present invention, there is also provided a method for isolating and culturing a single mesenchymal stem cell, comprising: a first culture step of culturing a cell population in a first culture medium, a collection step of collecting the cell population after the first culture step, an isolation step of isolating a single mesenchymal stem cell from the cell population after the collection step, and a second culture step of culturing the single mesenchymal stem cell after the isolation step in a second culture medium that does not comprise other mesenchymal stem cells, wherein the first culture medium and/or the second culture medium (for example, both the first culture medium and the second culture medium or any one of the first culture medium or the second culture medium) comprises human platelet lysate. More preferably, there is provided a method in which both the first culture medium and the second culture medium comprise human platelet lysate.

### Examples

The present invention will be specifically described below with reference to Examples. These Examples are for explaining the present invention, and do not limit the scope of the present invention.

### <Example 1: Isolation of single cell after FBS culture or after hPL culture>

### (Purpose)

The purpose is to culture a mesenchymal stem cell derived from a bone marrow or an amniotic membrane, in a culture medium comprising a fetal bovine serum (FBS) or human platelet lysate (hPL), isolate the cultured cell using a cell sorter, culture a single cell in each well in a 96-well plate, and measure the number of wells comprising a proliferating cell.

### (Methods)

### (1) FBS culture of bone marrow mesenchymal stem cell

A bone marrow fluid was collected from an adult donor with informed consent. An αMEM (Alpha Modification of Minimum Essential Medium Eagle) comprising a fetal bovine serum (FBS (Moregate, 599-04425)) at a final concentration of 10% and a bone marrow fluid were mixed at a ratio of 5:1, and seeded in a culture vessel. Then, while replacing the culture medium with a culture medium comprising FBS at a frequency of once every 3 to 4 days, the bone marrow mesenchymal stem cell was subjected to adherent culture until the resultant cells became subconfluent. Hereinafter, a method for culturing in a culture medium comprising FBS is referred to as "FBS culture", and the bone marrow mesenchymal stem cell was subcultured under the same conditions if necessary.

### (2) hPL culture of bone marrow mesenchymal stem cell

A bone marrow fluid was collected from an adult donor with informed consent. An αMEM comprising human platelet lysate (hPL (AventaCell, HPCFDCGLI50)) at a final concentration of 5% and a bone marrow fluid were mixed at a ratio of 5:1, and seeded in a culture vessel. Then, while replacing the culture medium with a culture medium comprising hPL at a frequency of once every 3 to 4 days, the bone marrow mesenchymal stem cell was subjected to adherent culture until the resultant cells became subconfluent. Hereinafter, a method for culturing in a culture medium comprising hPL is referred to as "hPL culture", and the bone marrow mesenchymal stem cell was subcultured under the same conditions if necessary.

### (3) FBS culture of amniotic membrane mesenchymal stem cell

A fetal membrane and a placenta which are fetal appendages were aseptically collected from a pregnant woman in an elective cesarean section case with informed consent. The obtained fetal membrane and placenta were accommodated in a sterile vat comprising a physiological saline, and an amniotic membrane was manually detached from a stump of the fetal membrane. The amniotic membrane was washed with a Hanks' balanced salt solution (free of Ca/Mg), and attached blood and a blood clot were removed.

The amniotic membrane comprising an epithelial cell layer and a mesenchymal stem cell layer was immersed in a Hanks' balanced salt solution (comprising Ca/Mg) comprising 480 PU/mL collagenase and 400 PU/mL dispase I, and the amniotic membrane was enzyme-treated by shaking and stirring under a condition of 50 rpm at 37°C for 90 minutes. The resultant solution after enzyme treatment was filtered through a nylon mesh having an opening of 95 µm to remove an undigested material of the amniotic membrane, and a cell suspension comprising an amniotic membrane mesenchymal stem cell was collected.

A cell population comprising the amniotic membrane mesenchymal stem cell was seeded in a culture vessel at a seeding density of 6,000 cells/cm². After cell seeding, adherent culture was performed in an αMEM comprising FBS at a final concentration of 10% until the cells became subconfluent. Then, the amniotic membrane mesenchymal stem cell was subcultured under the same conditions if necessary.

### (4) hPL culture of amniotic membrane mesenchymal stem cell

A fetal membrane and a placenta which are fetal appendages were aseptically collected from a pregnant woman in an elective cesarean section case with informed consent. The obtained fetal membrane and placenta were accommodated in a sterile vat comprising a physiological saline, and an amniotic membrane was manually detached from a stump of the fetal membrane. The amniotic membrane was washed with a Hanks' balanced salt solution (free of Ca/Mg), and attached blood and a blood clot were removed.

The amniotic membrane comprising an epithelial cell layer and a mesenchymal stem cell layer was immersed in a Hanks' balanced salt solution (comprising Ca/Mg) comprising 480 PU/mL collagenase and 400 PU/mL dispase **I,** and the amniotic membrane was enzyme-treated by rotational stirring using a rotator under a condition of 10 rpm at 37°C for 60 minutes. The resultant solution after enzyme treatment was filtered through a nylon mesh having an opening of 95 µm to remove an undigested material of the amniotic membrane, and a cell suspension comprising an amniotic membrane mesenchymal stem cell was collected.

A cell population comprising the amniotic membrane mesenchymal stem cell was seeded in a culture vessel at a seeding density of 1,400 cells/cm². After cell seeding, adherent culture was performed in an αMEM comprising hPL at a final concentration of 5% until the cells became subconfluent. After reaching subconfluency, the amniotic membrane mesenchymal stem cell was subcultured at a seeding density of 1,000 cells/cm², and cultured until the cells became subconfluent. Then, the amniotic membrane mesenchymal stem cell was subcultured under the same conditions if necessary.

### (5) Single cell cloning

Various mesenchymal stem cells cultured in the above (1) to (4) were detached using TrypLE (trademark) Select Enzyme (1X), and the cell concentration was adjusted to 1 × 10⁶ cells/mL with a phosphate-buffered physiological saline comprising 0.5% bovine serum albumin. A 7-AAD (7-Amino-Actinomycin D) staining solution was added to this cell suspension, and dead cells were stained.

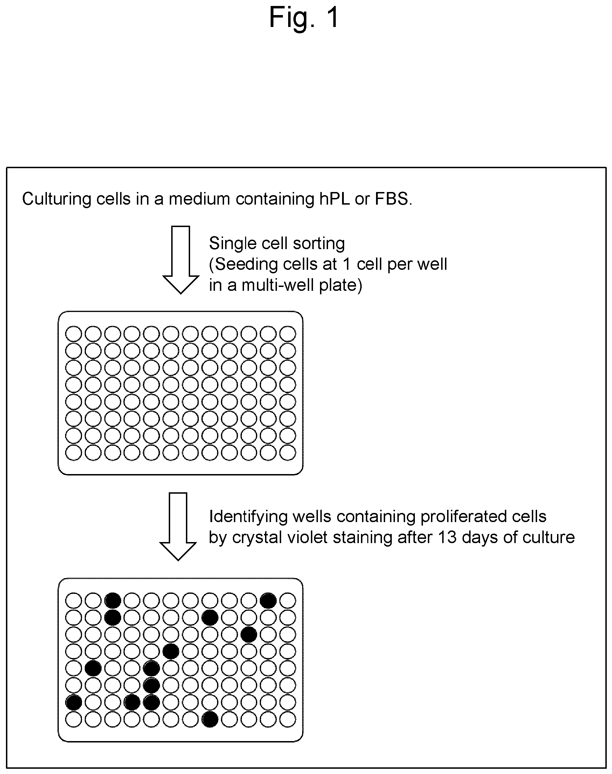

With the cell suspension used as a specimen, only living cells (7-AAD negative fraction) were sorted at 1 cell/well into five 96-well plates for each type of mesenchymal stem cell using a cell sorter MA900. A mesenchymal stem cell that entered each well by sorting was cultured in each well in a culture medium comprising human platelet lysate, all wells were stained with a crystal violet staining solution 13 days after sorting, and wells comprising proliferated cells were counted (Figure 1).

### (Results)

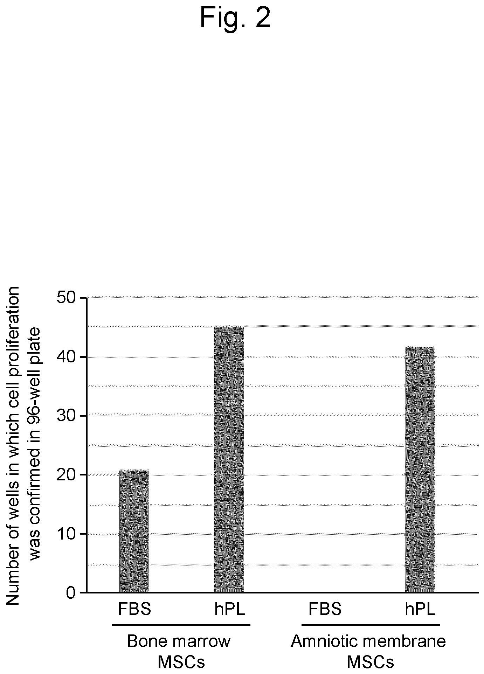

Figure 2 shows the result of measuring the number of wells in which cell proliferation was confirmed in 96 wells under each condition.

Under conditions where bone marrow mesenchymal stem cells (Bone marrow MSCs in the figure) were hPL-cultured, then subjected to single cell sorting, and further cultured, cell proliferation was observed in at least twice as many wells compared with conditions where single cell sorting and further culture were performed after FBS culture.

Additionally, under conditions where amniotic membrane mesenchymal stem cells (Amniotic membrane MSCs in the figure) were FBS-cultured, then subjected to single cell sorting, and further cultured, a well exhibiting cell proliferation was not confirmed at all, whereas under conditions where single cell sorting and further culture were performed after hPL culture, cell proliferation was observed in nearly half of all wells.

The above results indicate that a single cell of a mesenchymal stem cell having proliferative ability can be obtained with high efficiency by performing a culture in a culture medium comprising hPL before performing single cell sorting.

### <Example 2: FBS long-term subculture of mesenchymal stem cell>

### (Purpose)

The purpose is to subculture a bone marrow mesenchymal stem cell and an amniotic membrane mesenchymal stem cell over a long period of time without isolating the cells as single cells, and verify that a mesenchymal stem cell exhibits proliferative properties in a culture medium comprising FBS.

### (Methods and results)

The bone marrow mesenchymal stem cells acquired in (1) of Example 1 were repeatedly subcultured in an αMEM comprising FBS at a final concentration of 10%, and cultured until proliferation stopped.

Additionally, the amniotic membrane mesenchymal stem cells acquired in (3) of Example 1 were repeatedly subcultured in an αMEM comprising FBS at a final concentration of 10%, and cultured until proliferation stopped.

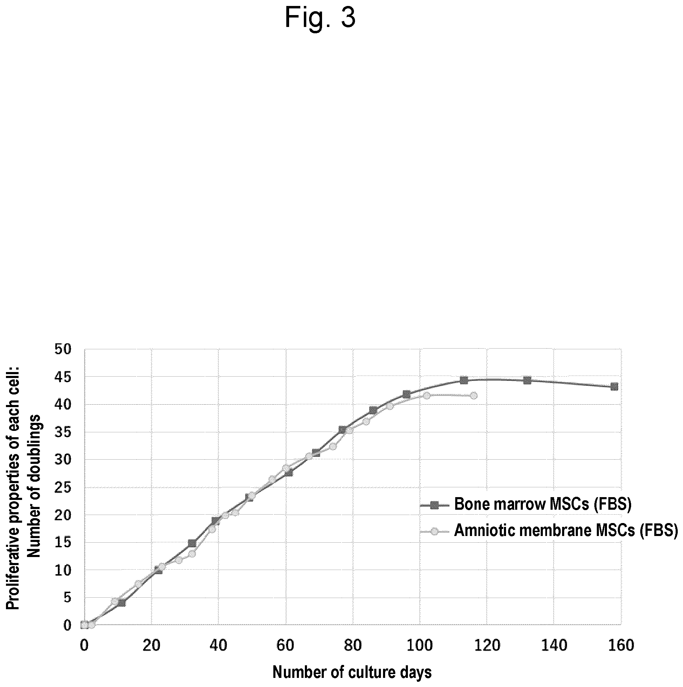

For the bone marrow mesenchymal stem cells and the amniotic membrane mesenchymal stem cells, the number of cells was measured before each passage during a long-term culture period. Figure 3 shows the result of calculating the number of doublings. Both the bone marrow mesenchymal stem cells and the amniotic membrane mesenchymal stem cells exhibited 40 to 50 doublings. From this result, it was observed that the bone marrow mesenchymal stem cell and the amniotic membrane mesenchymal stem cell exhibit normal proliferative properties in a culture medium comprising FBS.

From the result that normal proliferative properties were observed when long-term culture was performed in a culture medium comprising FBS without isolating single cells in the present Example, it is clear that in a case where the cells are cultured in the form of a cell population, proliferative properties are not impaired even in a culture medium comprising FBS. Accordingly, in a case where single cells were isolated and cultured after FBS culture in Example 1 above, the result that the amniotic membrane mesenchymal stem cells did not exhibit cell proliferation at all, and proliferative properties of the bone marrow mesenchymal stem cells were low is considered to be a phenomenon associated with single cell culture.

All publications, patents, and patent applications cited herein are incorporated herein by reference in their entirety.

## Claims

1. A method for isolating a single mesenchymal stem cell from a cell population comprising mesenchymal stem cells, comprising:
a first culture step of culturing the cell population in a first culture medium comprising human platelet lysate;
a collection step of collecting the cell population after the first culture step; and
an isolation step of isolating a single mesenchymal stem cell from the cell population after the collection step.

2. The method of claim 1, wherein the cell population is derived from amniotic membrane or bone marrow.

3. The method of claim 1, wherein the first culture medium comprises 3 v/v% or more of the human platelet lysate.

4. The method of claim 3, wherein the first culture medium comprises 3 to 20 v/v% of the human platelet lysate.

5. The method of claim 1, wherein the first culture medium further comprises heparin or a heparin substitute.

6. The method of claim 1, wherein the first culture medium is a basic culture medium or a serum-free culture medium.

7. The method of claim 1, wherein the collection step comprises collecting the cell population from the first culture medium.

8. The method of claim 1, wherein dead cells or living cells are labeled after the collection step, and only the living cells are isolated in the isolation step.

9. The method of claim 1, further comprising an introduction step of introducing a gene expression vector into the cell population before the isolation step.

10. The method of claim 9, wherein the gene expression vector comprises a marker gene.

11. The method of claim 9, wherein the introduction step is performed before the first culture step.

12. The method of claim 10, wherein the isolation step comprises isolating the single mesenchymal stem cell expressing a marker protein encoded by the marker gene, using the expression of the marker protein as a marker.

13. The method of claim 10, wherein the marker gene is an antibiotic resistance gene that confers resistance to an antibiotic.

14. The method of any one of claims 1 to 13, further comprising a second culture step of culturing the single mesenchymal stem cell after the isolation step in a second culture medium that does not comprise other mesenchymal stem cells.

15. The method of claim 14 referring to claim 13, wherein the second culture medium comprises the antibiotic.

16. A method for producing a single mesenchymal stem cell, comprising producing the single mesenchymal stem cell using the method of claim 1.

17. A method for producing a population of mesenchymal stem cells derived from a single mesenchymal stem cell, comprising producing the population of mesenchymal stem cells by proliferating the single mesenchymal stem cell in the second culture step using the method of claim 14.

18. A method for culturing a single mesenchymal stem cell, comprising:
a first culture step of culturing a cell population comprising mesenchymal stem cells in a first culture medium;
a collection step of collecting the cell population after the first culture step;
an isolation step of isolating a single mesenchymal stem cell from the cell population after the collection step; and
a second culture step of culturing the single mesenchymal stem cell after the isolation step in a second culture medium that does not comprise other mesenchymal stem cells,
wherein the second culture medium comprises human platelet lysate.
